# EUROPEAN PATENT SPECIFICATION

(11) **EP 0 891 318 B1**
(45) Date of publication and mention of the grant of the patent: **14.11.2001**
(21) Application number: 97901201.0
(22) Date of filing: 28.01.1997
(51) Int. Cl.: C07C 53/08, C07C 51/46, C07C 63/26, C07C 51/265

(54) **AZEOTROPIC DISTILLATION PROCESS**
AZEOTROPER DESTILLATIONSPROZESS
PROCEDE DE DISTILLATION AZEOTROPE

(30) Priority: 09.02.1996 GB 9602680
(43) Date of publication of application: 20.01.1999
(73) Proprietor: E.I. DU PONT DE NEMOURS AND COMPANY, Wilmington Delaware 19898 (US)
(72) Inventor: PARTEN, William, David, Tamebridge Stokesley North Yorkshire TS9 (GB)
(74) Representative: Jones, Alan John
(86) International application number: GB9700235
(87) International publication number: WO9729068

(56) References cited:
- WO-A-96/06065
- GB-A- 1 576 787
- US-A- 4 250 330
- US-A- 4 661 208

## Description

This invention relates to distillation processes and is particularly concerned with the recovery of entrainer compounds used in azeotropic distillation processes.

US-4250330 discloses a basic azeotropic distillation process for effecting separation of water from acetic acid, the entrainer being isobutyl acetate. US-4661208 discloses that energy may be saved by reheating a stream taken either from a decanter on an azeotropic column or a sidestream from the column and returning it to the column below the main feed. GB-1576787 discloses a scheme in which some of the organic phase from an azeotropic distillation column is refluxed into the centre of the column in order to reduce the time lag between seeing an effect in a control variable, the temperature in the middle of the column, and a manipulated variable, the amount of organic phase refluxed. The scheme is reported as being effective in the azeotropic separation of water from a number of organic compounds such as acetic acid and various alcohols.

Our prior International Patent Application No. PCT/GB95/01933 describes a heterogeneous azeotropic distillation process for effecting the separation of water from acetic acid so that acetic acid having a lower water content can be recovered from the base of the distillation column. In that patent application, reference is made to the use of the distillation process in connection with the production of aromatic acids (e.g. terephthalic acid) where the acetic acid and water feed stream is derived, at least in part, from a reactor in which the liquid phase oxidation of a precursor of the aromatic acid in admixture with acetic acid is effected in the presence of a catalyst system. In the case of terephthalic acid production, the precursor is paraxylene. In carrying out the reaction, water is produced and the heat of reaction is removed by withdrawing vapour and gases produced in the reaction and condensing those components which are condensable, mainly water and acetic acid along with other components including some of the precursor. A relatively water-lean, acetic acid-rich fraction of the condensate is recycled to the oxidation reactor while a relatively water-rich, acetic acid-lean fraction is fed to the azeotropic distillation column and, as described in International Patent Application No. PCT/GB95/01933, the precursor of the aromatic acid is recovered by withdrawing it as a purge from the column.

When the precursor is purged in this way, some entrainer is also removed from the distillation column. The purge can be recycled to the oxidation reactor and in the case of an alkyl acetate entrainer, at least part of the purged entrainer will then pass into the overheads vapour/gas stream from the reactor and, following condensation along with water, acetic acid and the precursor, will be fed back to the distillation column. Whilst conversion of the purged entrainer into other compounds will occur in the oxidation reactor, such compounds will not deleteriously affect the quality of the desired aromatic acid product to any significant extent provided a suitable entrainer is selected, e.g. n-propyl acetate. Nevertheless, because some conversion of the entrainer will take place, the consequent loss of entrainer will require continual addition of fresh entrainer to the distillation column

The present invention seeks to reduce loss of entrainer as a result of purging said precursor from the distillation column.

According to one aspect of the present invention there is provided a process for the azeotropic distillation of a feed stream containing an aliphatic carboxylic acid, a hydrocarbon and water to produce a bottoms product comprising aliphatic carboxylic acid having a lower water content than the feed stream, in which a purge stream containing the hydrocarbon, said aliphatic carboxylic acid, the entrainer and water is withdrawn from the azeotropic zone within the distillation column and the entrainer is recovered for recycle to the azeotropic zone of the column, recovery being effected in a stripping column operated to produce a bottoms product including the hydrocarbon as a major constituent and a tops product containing the entrainer as a major constituent.

The hydrocarbon is typically a compound which is oxidisable to produce an aromatic polycarboxylic acid; for example paraxylene which can be oxidised to produce terephthalic acid.

The aliphatic carboxylic acid is typically one which is used in the production of aromatic polycarboxylic acids by the liquid phase oxidation of a hydrocarbon precursor; for example, acetic acid which can be used in the liquid phase oxidation of xylenes to produce dicarboxylic acids such as terephthalic acid and isophthalic acid.

The entrainer is typically an acetate such as n-propyl acetate.

Where the hydrocarbon is one whose liquid phase activity coefficient is increased in the presence of said aliphatic carboxylic acid in such a way that the volatilities of the hydrocarbon and the entrainer tend to converge, preferably water is added to the stripping column to offset the convergence effect and thereby facilitate stripping of the entrainer from the hydrocarbon.

Preferably the stripping column is operated in such a way as to establish a temperature profile lengthwise of the column having a first lower temperature plateau at a higher location in the column, a second higher temperature plateau at a lower location in the column and an increasing temperature extending from said second plateau to the base of the stripping column.

Such a temperature profile may be established by control of the heat input to the stripping column so as to obtain the first plateau with a control temperature point between the two plateaus, and by addition of water to cause the second plateau to occur and to produce a further temperature rise in the base of the column. The two plateaus respectively indicate efficient removal of the entrainer from the hydrocarbon and, within the limitations of a stripping only column, efficient removal of the hydrocarbon from the entrainer.

According to a second aspect of the present invention there is provided a process for the production of an aromatic polycarboxylic acid comprising oxidising a precursor of the polycarboxylic acid in a liquid phase medium comprising a lower aliphatic carboxylic acid and in the presence of a heavy metal catalyst system, the oxidation being accompanied by the production of an overhead vapour stream comprising the aliphatic carboxylic acid, said precursor and water, condensing the overhead vapour stream to produce a liquid phase feed stream containing the aliphatic carboxylic acid, water and said precursor, distilling the feed stream in a column to produce a bottoms product containing the aliphatic carboxylic acid and a reduced amount of water, withdrawing a purge containing the precursor, the entrainer and water from the column, processing the purge to separate entrainer from the precursor, and recycling the separated entrainer to the distillation column.

In this way, loss of entrainer as a result of passage through the oxidation reaction can be substantially reduced or eliminated.

Preferably the feed stream containing the aliphatic carboxylic acid, water and said precursor is introduced into the column at a location above the lower limit of the azeotropic zone and the purge is withdrawn from the column in the region of the location at which said feed stream is introduced, the point of withdrawal conveniently being at a location above the point of introduction of the feed stream.

As used throughout this specification, "azeotropic zone" refers to that region of the distillation column where the concentration of the entrainer in the combined liquid phases is at least 0.1% by weight.

In practice, the point at which the feed stream is introduced into the column will be consistent with the need to minimise the concentration of the aliphatic acid in the tops product withdrawn at the upper end of the column and will be somewhat closer to the lower limit of the azeotropic zone than to the top of the column.

Typically the feed stream subjected to azeotropic distillation has a water content in excess of 20%, e.g. up to 60%, by weight based on the combined weight of the aliphatic carboxylic acid and water in the feed stream and a bottoms product is obtained which is substantially free of entrainer, typically containing an amount of water within the range 2 to 12% by weight based on the combined weight of the aliphatic carboxylic acid and water in the bottoms product.

Where the water content of the feed stream subjected to azeotropic distillation has a relatively low water content, e.g. from 20 to 40% by weight based on the combined weight of the aliphatic carboxylic acid and water in the feed stream, the distillation column is preferably operated with single organic phase reflux comprising said entrainer. As disclosed in our prior International Application No. PCT/GB 95/01933 (the entire disclosure of which is incorporated herein by this reference), the water content of the feed stream may be from 20 to 30% (e.g. 23 to 27%) by weight based on the combined weight of the aliphatic carboxylic acid and water in the feed stream.

In a preferred embodiment of the invention separation of entrainer from said precursor is carried out in a stripping column in such a way that substantially all of the entrainer is vaporised and recovered as the tops product while some of the precursor is recovered at the base of the stripping column.

Where the precursor and the entrainer have comparable volatilities (especially in the presence of aliphatic carboxylic acid which tends to be withdrawn in the purge stream from the distillation column), with consequent difficulty in separating these two components from each other, we have found that the addition of water or an aqueous stream to the stripping column, e.g. by addition to the purge stream, significantly enhances separation. The water/aqueous stream may be introduced into the stripping column as a liquid phase or as a gaseous phase (e.g. steam injection).

Preferably the stripping column is operated so that some of the precursor together with aliphatic carboxylic acid and/or water is recovered at the base of the stripping column. In practice, the stripping column will be operated so that the amount of precursor recovered at the base of the stripping column is sufficient to maintain the paraxylene content in the azeotropic distillation column within tolerable limits.

The recovered entrainer is conveniently reintroduced into the distillation column, preferably as a vapour, at the same or substantially the same level at which the purge is withdrawn.

The entrainer is preferably an acetate compound such as an alkyl acetate, n-propyl acetate being particularly preferred especially in the case where the aliphatic carboxylic acid comprises acetic acid and said precursor comprises paraxylene or an isomer thereof.

The invention will now be described by way of example only with reference to use of the process for recovering paraxylene in the course of using n-propyl acetate as entrainer to effect azeotropic distillation of a feed stream derived from the liquid phase oxidation of paraxylene to produce terephthalic acid.

The oxidation is carried out in a reactor in which the liquid phase medium comprises paraxylene, acetic acid solvent, some water and a brominated catalyst system comprising cobalt and manganese compounds. Such an oxidation process is described in our prior EP-A-498501 and EP-A-502628, the disclosures of which are incorporated herein by this reference. The oxidation process results in the generation of a reactor overhead vapour comprising mainly acetic acid and water of reaction together with other compounds such as methyl acetate and paraxylene. This overhead vapour is withdrawn from the reactor and is partially condensed in an overheads condenser system to produce liquid phase aqueous acetic acid components, a relatively water-lean, acetic acid-rich component which is returned to the reactor as a reflux and a relatively water-rich, acetic acid-lean component which is passed to the distillation column. The latter component contains a water content of the order of 20 to 30% (typically 25 to 28%) by weight based on the combined acetic acid and water content of the stream. The aqueous acetic acid stream usually also contains some paraxylene and methyl acetate.

Azeotropic distillation is employed to produce a bottoms product comprising acetic acid with a reduced water content (typically 5% by weight based on the combined acetic acid/water content) whereby the water content in the oxidation reactor can be regulated by removing excess water and returning a residual amount together with the recycled acetic acid. The lower reflux ratios that can be employed through use of high boiling point entrainers such as n-butyl acetate make such entrainers the logical choice for the azeotropic distillation, especially where the intention is to make more effective use of the significant waste heat generated in the oxidation reaction or to operate the oxidation process at reduced pressure with attendant reduced energy input requirements. However, the water content present in the overheads aqueous acetic acid stream and that present in the acetic acid product derived from the azeotropic distillation are such that high boiling point entrainers require special steps to be taken to prevent slippage of the entrainer into the bottoms product; for instance, operation with a combined organic phase and aqueous phase reflux and/or processing of the reactor overheads stream to increase the water content of the feed to a level effective to strip out substantially all of the entrainer above the point of withdrawal of the bottoms product from the distillation column.

These complications can be avoided by limiting the processing of the overheads aqueous acetic acid stream coupled with operating the distillation process with a single organic phase reflux and so that the acetic acid bottoms product is substantially entrainer free and contains the requisite level of water consistent with recycle to the oxidation reactor. This is achieved by using a relatively low boiling point entrainer such as n-propyl acetate, iso-butyl acetate or a compound which has an intermediate boiling point, is compatible with the desired separation and forms a heterogeneous azeotrope with water. By "limiting processing of the reactor overheads aqueous acetic acid stream" we mean that the vapour phase reactor overheads are subjected to condensation processes without taking special additional steps to increase the water content by way of additional rectification equipment.

The feed stream supplied to the distillation column contains some paraxylene. As disclosed in prior International Patent Application No. PCT/GB 95/01933, we have found that the use of an alkyl acetate with a relatively low boiling point, e.g. n-propyl acetate, compared with n-butyl acetate has the effect of causing paraxylene to follow a concentration profile, lengthwise of the column, such that paraxylene concentration increases markedly in the region of the point of introduction of the feed stream thus allowing paraxylene to be purged from the column in significant amounts at a single location for recycle to the oxidation reactor. This allows paraxylene to be removed in the course of the distillation rather than having to resort to other means such as removal prior to introduction of the feed stream into the distillation column. However, purging of paraxylene is inevitably accompanied by draw off of the entrainer and acetic acid. Draw off of the acetic acid is not a problem since it can be recycled to the reactor together with the paraxylene. Likewise, draw off of the entrainer is not necessarily a problem because it too can be passed to the reactor together with the paraxylene and acetic acid in the purge stream. Whilst a substantial proportion of the entrainer will find its way back to the distillation column, significant quantities of the entrainer will nevertheless be consumed by conversion to other compounds as a result of being fed to the reactor and continual replenishment of the entrainer lost in this manner is therefore necessary.

In order to minimise replenishment of entrainer, the purge from the distillation column is passed to a stripping column. With specific reference to n-propyl acetate (npa) as the entrainer, the paraxylene purge stream from the azeotropic distillation column will contain primarily npa, paraxylene, acetic acid and water. Of these components, the aim is to return npa to the azeotropic distillation column and purge some paraxylene back to the oxidation reactor while the water and acetic acid, being present in small quantities compared to the main plant streams, may be returned to the distillation column and/or to the oxidation reactor and/or otherwise disposed of. If the vapour liquid properties of the system are examined:
npa boils at 102°C and forms a two liquid phase azeotrope with water at 82°C;
paraxylene boils at 137°C, forms a homogeneous azeotrope with acetic acid at 115°C and a two liquid phase azeotrope with water at 92°C; and
acetic acid boils at 118°C; and
water boils at 100°C.

The purge stream from the azeotropic distillation column will have a composition depending on the refluxes used, feed compositions and the purging history but will typically have npa in excess of the paraxylene with the combined content of the npa and paraxylene forming up to 75% by weight of the stream. The purge stream will also tend to have a low water content and the remainder of the stream will be acetic acid. In a stripping column the water will be quickly removed in the top few stages because of low boiling azeotropes with npa and paraxylene. The required separation is then between npa and paraxylene but the relative volatility of one with respect to the other will depend on the amount of acetic acid remaining. With relatively high acetic acid concentrations, the volatilities of npa and paraxylene are similar due to the high activity coefficient of paraxylene caused by the presence of acetic acid. Experimentally we have found that there is little difference in the boiling point of mixtures with widely different npa to paraxylene ratios once there was a significant amount of acetic acid present, e.g. 25% or more.

One way of dealing with the similar volatilities of npa and paraxylene under these conditions is to reduce the acetic acid content of the purge stream. However, an alternative and preferred way involves the addition of water into the process. The effect of adding water as such or in the form of an aqueous stream is to raise the activity coefficients of both the npa and the paraxylene but the effect on the npa is greater because the activity coefficient of the paraxylene has already been raised by the presence of acetic acid. Consequently the relative volatility of npa to paraxylene is increased and it is possible to remove the npa substantially completely, leaving substantial amounts of the paraxylene to be removed from the base of the column. The npa which is removed (along with water, acetic acid and some paraxylene carried over at the top of the stripping column) is returned, preferably as a vapour, to the azeotropic distillation column and to a location at or near the location from which the purge is withdrawn.

The addition of extra water to the stripping column allows a separation of npa from paraxylene to occur in the upper portions of the stripping column whilst some water may be removed from the base of the column if so desired. In this situation, water is removed from the top of the stripping column as an azeotrope with npa and/or paraxylene. Without the added water, there would be insufficient water to remove substantially all of the npa as an azeotrope which would then lead to a situation where separation of npa from paraxylene would have to be carried out In the presence of acetic acid with no water present. By introducing additional water, it is possible to ensure that substantially all of the npa is taken overhead as the azeotrope with water while leaving sufficient water in the column to enhance the npa/paraxylene separation. Such additional water will leave the column via the base. In a preferred embodiment of the scheme the following operation is followed.
1) A liquid purge from just above the main feed stream to the azeotropic distillation column is fed to the stripping column.
2) A water or an aqueous stream is co-fed to the stripping column. The aqueous stream may for instance be derived from a dilute water/acetic acid stream produced in the course of operating the terephthalic acid production plant, for instance a stream resulting from scrubbing acetic acid vapour from an offgas using water as the scrubbing medium. Ordinarily such water/acetic acid streams would be fed to the azeotropic distillation column but one or more of these streams may instead be used as a source of the extra water fed to the stripping column rather than using fresh water.
3) Heat is applied to the base of the stripping column such that there is a relatively constant temperature down the first few stages, e.g. 5 theoretical stages, which will be in the range 85 to 90°C with typical purge stream compositions and pressures at or close to atmospheric, but may be higher. This indicates the penetration of npa down the column. The temperature is allowed to rise across the next few stages, by appropriate regulation of the reboiler steam rate, to reach a second plateau running at a temperature which is higher, for example by 3 to 8°C, indicating removal of substantially all of the npa and a constant composition acetic acid/paraxylene/water mixture present in the column.
4) The water or aqueous stream fed to the top of the column is regulated to ensure the existence of both of these temperature plateaus and to give a fixed rise in temperature at the base of the column. Typically a temperature rise of 10°C is appropriate from the second plateau to the base of the column. A larger rise in temperature up to the azeotropic temperature for the acetic acid/paraxylene azeotrope will give rise to greater recycle of paraxylene to the azeotropic distillation column and a lower rise will lead to a higher water content in the recycled paraxylene stream back to the oxidation reactor. By appropriate control of the additional water introduced into the stripping process, it is possible to maintain a temperature in the base of the column intermediate the paraxylene/water azeotropic temperature and the paraxylenelacetic acid azeotropic temperature such that a suitable compromise is arrived at between recycle of paraxylene with npa to the azeotropic distillation column and the amount of water present in the paraxylene recycled to the oxidation reactor.

Referring now to the drawings:

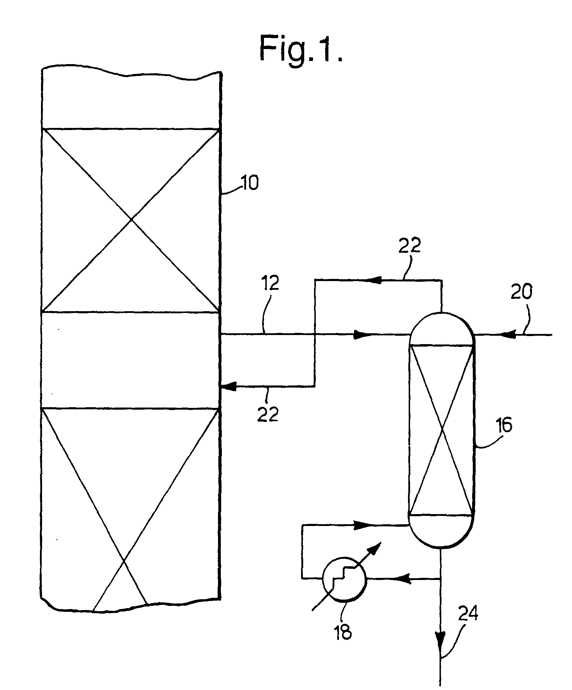

Figure 1 illustrates diagrammatically processing of the purge stream withdrawn from the azeotropic distillation column by means of a stripping column; and
Figure 2 illustrates qualitatively the temperature profile along the stripping column.

The azeotropic distillation column is depicted by reference 10 and may be arranged to operate in the manner disclosed in our prior International Patent Application No. PCT/GB 95/01933. A purge stream comprising paraxylene, acetic acid, n-propyl acetate and water is taken via line 12 and is passed to the top of a stripping column 16 equipped with reboiler 18. The reboiler 18 may for instance use steam as the heating source. The stripping column 16 may be a frayed column or it may be a packed column. Additional water is supplied to the top of the stripping column 16 via line 20, the additional water conveniently being in the form of an aqueous stream of acetic acid derived from elsewhere in the terephthalic acid production plant. The tops product containing n-propyl acetate, some paraxylene and water is returned, preferably while still in the vapour phase, to the azeotropic distillation column 10 via line 22 at or near the same location as the purge withdrawal via line 12. The bottoms product containing some paraxylene, some water and acetic acid is removed via line 24 and can be recycled back to the oxidation reactor (not shown).

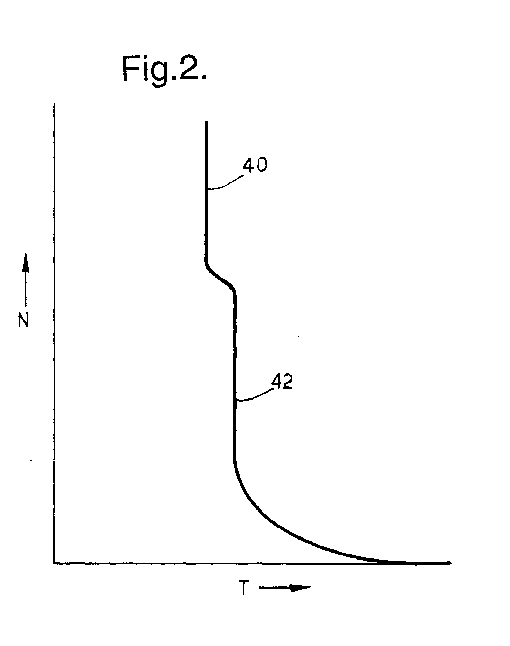

Referring to Figure 2, the temperature profile established within the stripping column 16 is shown in qualitative terms, the abscissa of the graph representing temperature T and the ordinate representing tray number N from the base of the stripping column. It will be seen that the temperature profile has a first plateau 40 where the temperature is substantially constant, e.g. at 87°C, and a second plateau 42 where the temperature is substantially constant, e.g. at 92°C. At the lower section of the column, the temperature increases progressively, e.g. from 92°C to 108°C. The temperatures mentioned here are only illustrative and may be higher or lower. An example illustrating the invention is given below.

### EXAMPLE

An Oldershaw column having 40 stages was supplied with a feed to stage 11 (counted from the top of the column), the feed comprising mixed organic and aqueous phase feeds, preheated to 70°C, to simulate the net effect of supplying an organic phase purge from the azeotropic distillation and feed of additional water to the stripping column. The organic feed comprised npa and paraxylene with a npa fraction by weight of 0.5. The aqueous phase feed constituted the previously mentioned aqueous addition and comprised acetic acid and water with an acetic acid fraction by weight of 0.6. Overheads vapours were condensed and retained as a tops product with no reflux being returned to the column. Without reflux, the column effectively operated as a thirty tray column.

The column was operated to obtain a profile with two plateaus as illustrated in Figure 2, with an upper plateau at 93°C and a second plateau at a temperature of 96°C. The second plateau extended from stage number 10 to about stage number 18 and was succeeded by a progressively rising temperature profile in the lower region of the column which reached a temperature of 118°C in the region of the reboller. Once steady column operation was secured, the bottoms product was found to comprise, in parts by weight, about 0.25 paraxylene, about 0.01 n-propyl acetate and balance acetic acid. In other words, substantially all of the npa present in the organic feed to the column was eliminated in the bottoms product, which consisted almost exclusively of acetic acid and paraxylene.

In the above example the recovery of npa from paraxylene in the presence of acetic acid has been described. Other, higher bolling, entrainers are sometimes used in the dehydration of acetic acid such as isobutyl and normal butyl acetates. Although we do not exclude the use of these entrainers, their separation from paraxylene would be harder to achieve than in the case of n-propyl acetate. This is because their higher boiling points make separation of the entrainer from the paraxylenelacetic acid azeotrope more difficult necessitating the need for the purge stream from the azeotropic distillation column to be largely acetic acid free. However, if the purge stream is reduced in acetic acid content, the separation of a higher boiling entrainer such as n-butyl acetate from paraxylene can be further enhanced in a stripping column by the addition of water to reduce the effect of the interaction between any residual acetic acid and paraxylene on the relative volatility of the entrainer to paraxylene.

## Claims

1. A process for the azeotropic distillation of water from a feed stream containing acetic acid, and a hydrocarbon, which is oxidizable to produce an aromatic polycarboxylic acid, using an alkyl acetate selected from isobutyl acetate and n-propyl acetate as an entrainer to produce a bottoms product comprising acetic acid having a lower water content than the feed stream, **characterised in that** a purge stream (12) containing the hydrocarbon, acetic acid, entrainer and water is withdrawn from the azeotropic zone of the distillation column (10) and introduced, optionally with a source of additional water (20), into a stripping column (16) operated to produce a bottoms product (24) containing the hydrocarbon as a major constituent and a tops product (22) containing the entrainer as a major constituent, and the entrainer is returned to the azeotropic zone of the distillation column.

2. A process as claimed in claim 1 in which the hydrocarbon is paraxylene.

3. A process as claimed in claim 2 further **characterized in that** the feed stream containing acetic acid, water, entrainer and precursor is introduced into the azeotropic column (10) at a location above the lower limit of the azeotropic zone, and the purge (12) is withdrawn from the azeotropic column (10) in the region of the location at which said feed stream is introduced.

4. A process as claimed in claim 3 in which the point of withdrawal of the purge stream (12) is at a location above the point of introduction of the feed stream.

5. A process as claimed in claim 3 or claim 4 in which the feed stream subjected to azeotropic distillation has a water content in excess of 20%.

6. A process as claimed in any one of claims 3 to 5 in which the bottoms product (24) obtained from the azeotropic distillation column (10) is substantially free of entrainer and contains an amount of water within the range 2 to 12% by weight based on the combined weight of the aliphatic carboxylic acid and water in the bottoms product (24).

7. A process as claimed in any one of claims 3 to 6 in which the water content of the feed stream subjected to azeotropic distillation has a water content of 20 to 40% by weight based on the combined weight of the acetic acid and water in the feed stream.

8. A process as claimed in claim 7 in which the azeotropic distillation column (10) is operated with single organic phase reflux comprising said alkyl acetate entrainer.

9. A process as claimed in any one of the preceding claims further **characterized by** operating the stripping column (16) to establish a temperature profile lengthwise of the column having a first lower temperature plateau at a higher location in the column, a second higher temperature plateau at a lower location in the column and an increasing temperature extending from said second plateau to the base of the stripping column.

## Patentansprüche

1. Verfahren für die azeotrope Destillation von Wasser aus einem Beschickungsstrom, enthaltend Essigsäure und einen Kohlenwasserstoff, der oxidierbar ist, unter Herstellen einer aromatischen Polycarbonsäure, unter Verwenden eines Alkylacetats, ausgewählt aus Isobutylacetat und n-Propylacetat als ein Schlepper, unter Herstellen eines Bodenprodukts, umfassend Essigsäure mit einem niedrigeren Wassergehalt als der Beschickungsstrom, **dadurch gekennzeichnet, daß** ein Spülstrom (12), enthaltend den Kohlenwasserstoff, Essigsäure, Schlepper und Wasser, aus der azeotropen Zone der Destillationssäule (10) abgezogen wird und eingeführt wird, wahlfrei mit einer Quelle von zusätzlichem Wasser (20), in eine Abtriebssäule (16), betrieben, um ein Bodenprodukt (24), das den Kohlenwasserstoff als einen Hauptbestandteil enthält, und ein Kopfprodukt (22), das den Schlepper als einen Hauptbestandteil enthält, herzustellen, und der Schlepper wird zu der azeotropen Zone der Destillationssäule zurückgeführt.

2. Verfahren nach Anspruch 1, bei dem der Kohlenwasserstoff Paraxylol ist.

3. Verfahren nach Anspruch 2, ferner **dadurch gekennzeichnet, daß** der Beschickungsstrom, der Essigsäure, Wasser, Schlepper und Vorläufer enthält, in die azeotrope Säule (10) an einer Stelle über der unteren Grenze der azeotropen Zone eingeführt wird, und der Spülstrom (12) wird aus der azeotropen Säule (10) in dem Bereich der Stelle abgezogen, bei der der Beschickungsstrom eingeführt wird.

4. Verfahren nach Anspruch 3, bei dem der Punkt des Abziehens des Spülstroms (12) an einer Stelle oberhalb des Einführungspunkts des Beschickungsstroms ist.

5. Verfahren nach Anspruch 3 oder Anspruch 4, wobei der Beschickungsstrom, der azeotroper Destillation ausgesetzt wird, einen Wassergehalt im Überschuß von 20% hat.

6. Verfahren nach einem der Ansprüche 3 bis 5, bei dem das Bodenprodukt (24), erhalten aus der azeotropen Destillationssäule (10), im wesentlichen frei von Schlepper ist und eine Wassermenge in dem Bereich von 2 bis 12 Gew.%, basierend auf dem kombinierten Gewicht der aliphatischen Carbonsäure und Wasser in dem Bodenprodukt (24), enthält.

7. Verfahren nach einem der Ansprüche 3 bis 6, bei dem der Wassergehalt des azeotroper Destillation ausgesetzten Beschickungsstroms einen Wassergehalt von 20 bis 40 Gew.%, basierend auf dem kombinierten Gewicht der Essigsäure und Wasser in dem Beschickungsstrom, hat.

8. Verfahren nach Anspruch 7, bei dem die azeotrope Destillationssäule (10) mit einem einzelnen organischen Phasenrückfluß, umfassend den Alkylacetatschlepper, betrieben wird.

9. Verfahren nach einem der vorhergehenden Ansprüche, ferner **gekennzeichnet durch** Betreiben der Abtriebssäule (16) unter Festlegen eines Temperaturprofils längs der Säule mit einem ersten niedrigeren Temperaturplateau an einer höheren Stelle in der Säule, einem zweiten höheren Temperaturplateau an einer unteren Stelle in der Säule und einer ansteigenden Temperatur, die sich von dem zweiten Plateau zu der Basis der Abtriebssäule erstreckt.

## Revendications

1. Procédé pour la distillation azéotrope d'eau à partir d'un courant d'alimentation contenant de l'acide acétique et un hydrocarbure, qui est oxydable pour produire un acide polycarboxylique aromatique, en utilisant un acétate d'alkyle choisi parmi de l'acétate d'isobutyle et de l'acétate de n-propyle en tant qu'agent entraînant pour obtenir un produit résidu de distillation comprenant de l'acide acétique présentant une teneur en eau inférieure à celle du courant d'alimentation, **caractérisé en ce qu'**un courant de purge (12) contenant l'hydrocarbure, l'acide acétique, l'agent entraînant et l'eau est retiré à partir de la zone azéotrope de la colonne de distillation (10) et introduit, éventuellement avec une source d'eau supplémentaire (20), dans une colonne de séparation (16) qui fonctionnait pour donner un produit résidu de distillation (24) contenant l'hydrocarbure comme constituant majeur et un produit fraction de tête (22) contenant l'agent entraînant comme constituant majeur et l'agent entraînant est retourné vers la zone azéotrope de la colonne de distillation.

2. Procédé suivant la revendication 1, dans lequel l'hydrocarbure est du paraxylène.

3. Procédé suivant la revendication 2, **caractérisé en outre en ce que** le courant d'alimentation contenant l'acide acétique, l'eau, l'agent entraînant et le précurseur est introduit dans la colonne azéotrope (10) à un endroit au-dessus de la limite inférieure de la zone azéotrope et la purge (12) est retirée de la colonne azéotrope (10) dans la région de l'endroit où ledit courant d'alimentation est introduit.

4. Procédé suivant la revendication 3, dans lequel le point de retrait du courant de purge (12) se trouve à un endroit au-dessus du point d'introduction du courant d'alimentation.

5. Procédé suivant la revendication 3 ou la revendication 4, dans lequel le courant d'alimentation soumis à une distillation azéotrope présente une teneur en eau dépassant 20%.

6. Procédé suivant l'une quelconque des revendications 3 à 5, dans lequel le produit résidu de distillation (24) obtenu à partir de la colonne de distillation azéotrope (10) est essentiellement exempt d'agent entraînant et contient une quantité d'eau dans l'intervalle de 2 à 12% en poids sur la base du poids combiné de l'acide carboxylique aliphatique et de l'eau dans le produit résidu de distillation (24).

7. Procédé suivant l'une quelconque des revendications 3 à 6, dans lequel la teneur en eau du courant d'alimentation soumis à une distillation azéotrope est une teneur en eau de 20 à 40% en poids sur la base du poids combiné de l'acide acétique et de l'eau dans le courant d'alimentation.

8. Procédé suivant la revendication 7, dans lequel la colonne de distillation azéotrope (10) fonctionnait avec un seul reflux de phase organique comprenant ledit agent entraînant d'acétate d'alkyle.

9. Procédé suivant l'une quelconque des revendications précédentes, **caractérisé en outre par** le fonctionnement de la colonne de séparation (16) pour établir un profil de température sur la longueur de la colonne présentant un premier plateau de température inférieure à un endroit plus haut dans la colonne, un deuxième plateau de température supérieure à un endroit plus bas dans la colonne et une température croissante s'étendant dudit deuxième plateau à la base de la colonne de séparation.
